# FASCICULE DE BREVET EUROPEEN

(11) **EP 1 116 493 B1**
(45) Date de publication et mention de la délivrance du brevet: **14.09.2005**
(21) Numéro de dépôt: 01400047.5
(22) Date de dépôt: 10.01.2001
(51) Int. Cl.: A61M 25/06, A61M 5/32

(54) **Dispositif anti-pique pour manipuler avec sécurité une aiguille d'injection**
Antistechvorrichtung zur sicheren Handhabung einer Injektionsnadel
Anti-stick device for secure handling of an injection needle

(30) Priorité: 12.01.2000 FR 0000330
(43) Date de publication de la demande: 18.07.2001
(73) Titulaire: VYGON, F-95440 Ecouen (FR)
(72) Inventeur: Huet, Jean-Max, 92110 Clichy (FR)
(74) Mandataire: Schrimpf, Robert

(56) Documents cités:
- US-A- 5 531 704
- US-A- 5 951 522

## Description

L'invention concerne un dispositif anti-pique pour manipuler avec sécurité une aiguille d'injection dans le domaine médical.

On connaît un dispositif (US 5,531,704) tel que défini dans le préambule de la revendication 1.

Un but de l'invention est de fournir un dispositif peu onéreux, facile à fabriquer et à manipuler, convenant à un usage unique.

Ce dispositif est tel que défini dans la revendication 1.

Dans des modes de réalisation préfères, le dispositif présente une ou plusieurs des caractéristiques additionnelles suivantes :
- la plaquette de base porte des ailettes latérales qui facilitent son application sur la peau ;
- la plaquette porte-aiguille présente une gorge pour recevoir une branche de l'aiguille qui forme un coude avec ladite partie de l'aiguille.

On décrira ci-après un mode de réalisation d'un dispositif conforme à l'invention, donné essentiellement à titre d'exemple non limitatif, en référence aux figures du dessin joint sur lequel :
- la figure 1 est une vue à plat d'un flan à mettre en volume pour constituer le dispositif de l'invention
- les figures 2 à 6 représentent des phases successives de la mise en volume du flanc de la figure 1 ;
- les figures 7 et 8 représentent deux phases de l'opération de mise en place du dispositif et
- les figures 9 et 10 représentent deux phases de l'opération d'extraction de l'aiguille hors de la peau.

Le dispositif représenté sur les figures est constitué à partir d'une paroi unique, par exemple, une feuille semi-rigide venue de découpage d'un flan en matériau de synthèse.

Cette paroi unique présente des lignes d'affaiblissement (1 à 5) constituant cinq lignes de pliages parallèles divisant la paroi en six panneaux successifs (A,B,C,D,E,F) ; les panneaux (F) et (D) constituent respectivement une plaquette porte-aiguille et une plaquette de base ; les panneaux (B), (C) et (E) constituent des panneaux de liaison entre ces deux plaquettes et le panneau (A) constitue un panneau apte à se fixer, par collage ou par soudage, à la plaquette porte-aiguille (F).

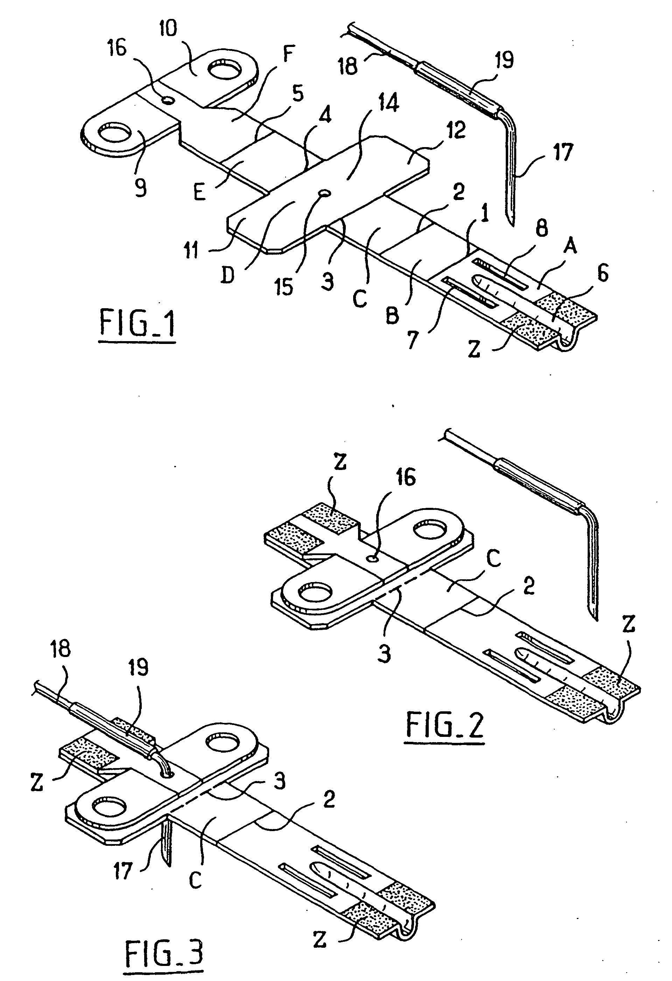

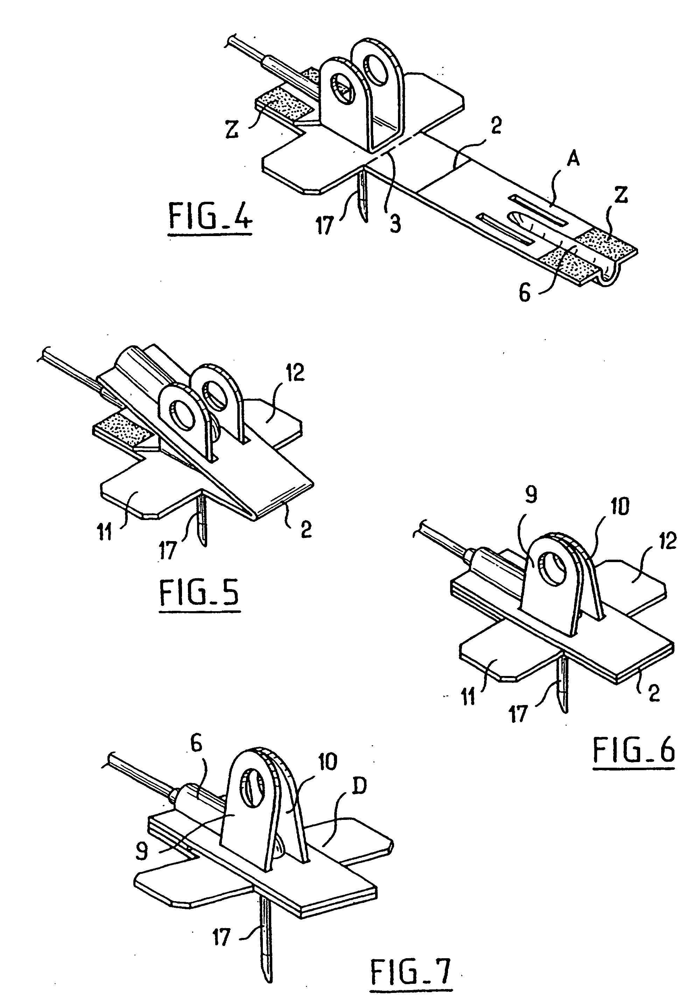

L'un (A) des panneaux d'extrémité de la succession de panneaux mis à plat présente deux lumières allongées parallèles (7,8) et l'autre panneau (F) de la succession de panneaux mis à plat présente deux oreilles latérales (9,10) aptes à être pliées à l'équerre avec le panneau, en vis-à-vis l'une de l'autre (figure 4), et à être introduites dans les lumières du panneau (A) lorsque ces deux panneaux sont appliqués l'un contre l'autre (figure 5) ; ces deux panneaux sont maintenus réunis par collage ou par soudage. On a représenté en grisé sur les figures 1 à 5 les zones (Z) de ces panneaux qui sont ainsi fixées l'une à l'autre.

Sur la figure 2, le tronçon d'extrémité (F) a été plié autour de la ligne (5) mais l'autre tronçon d'extrémité (A) n'a pas encore été plié.

Sur la figure 5, le tronçon d'extrémité (A) est en cours de pliage autour de la ligne (2) et n'est pas encore totalement rabattu contre le tronçon central (D) ; les deux oreilles (9,10) ont été engagées dans les lumières (7,8).

Dans la réalisation représentée, le tronçon central présente deux ailettes latérales (11,12) disposées en sorte que les oreilles (9,10) du tronçon d'extrémité (F) viennent s'appliquer sur ces ailettes lorsque ce tronçon est rabattu contre le tronçon central, et que l'épaisseur de la partie centrale (13) du tronçon d'extrémité (F) s'ajoute à l'épaisseur de la partie centrale (14) du tronçon central (D).

Ces deux parties centrales (13,14) des tronçons (D) et (F) sont munies de perforations (15,16) qui serviront au passage de la branche (17) de l'aiguille qui doit pénétrer dans le corps et cette double épaisseur contribue à un bon maintien de l'aiguille pendant la ponction.

Dans le cas représenté (figure 1) cette aiguille est coudée et présente une branche distale (17) perforante et une branche proximale (18) d'alimentation ; un tronçon de gaine (19) sur la branche proximale, à proximité du coude, facilite sa préhension pour sa mise en place dans le dispositif.

L'aiguille est mise en place et tenue dans le dispositif par introduction de sa branche distale perforante (17) dans les perforations (15,16) superposées (fig.3) et rabattement du panneau d'extrémité (A) sur la branche proximale (18) de l'aiguille (figures 5 et 6). Avantageusement, ce panneau d'extrémité (A) présente sur sa face qui sera tournée vers l'aiguille une gorge (6) dans laquelle se loge le tronçon de gaine (19) et le coude de l'aiguille.

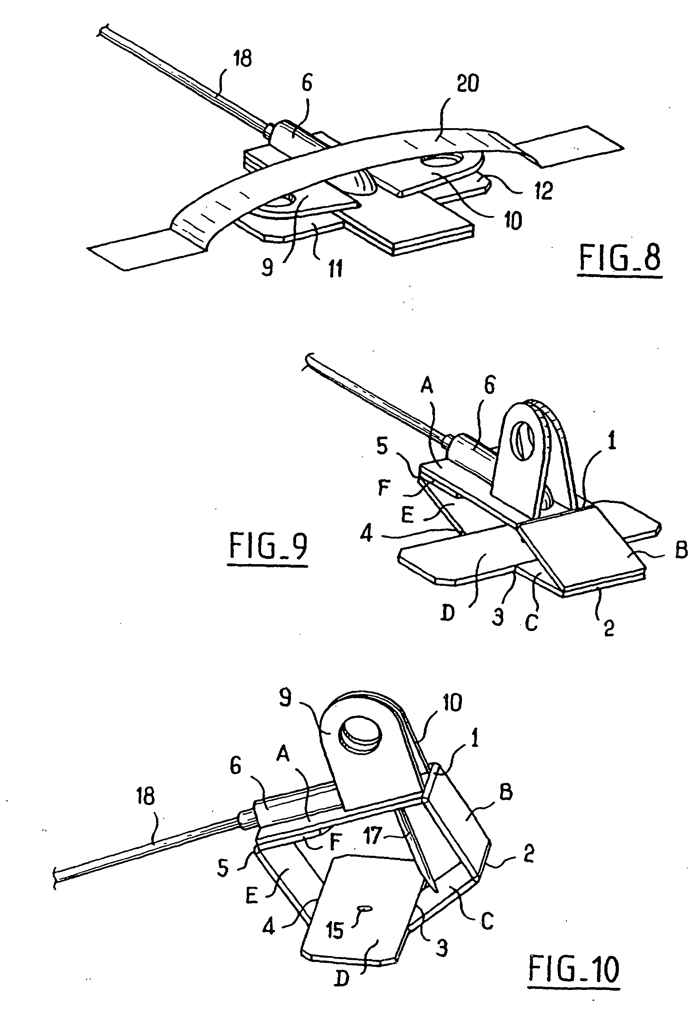

La mise en place du dispositif s'obtient en poussant le dispositif tenu par les oreilles (9,10) (figure 7) pour perforer la peau au moyen de la branche distale (17) de l'aiguille jusqu'à ce que le panneau central (D) du dispositif soit appliqué contre la peau ; les deux oreilles (9,10) sont ensuite rabattues à plat sur les pattes latérales (11,12) et un ruban adhésif (20) est appliqué sur le dispositif ainsi aplati ainsi que sur la peau pour maintenir en place l'aiguille et le dispositif (figure 8).

Pour extraire l'aiguille, l'opérateur ôte le ruban adhésif, redresse les oreilles (9,10) et les tire à lui d'une main pour extraire l'aiguille de la peau tout en maintenant avec son autre main le tronçon central (D) appliqué contre la peau (figure 9). Le dispositif se déforme grâce aux lignes de pliage au fur et à mesure de l'extraction en sorte que la branche perforante (17) de l'aiguille reste en permanence à l'intérieur du pentagone constitué par les panneaux (A) à (E) du dispositif (figure 10).

L'invention n'est pas limitée à ce mode de réalisation. En particulier, elle n'est pas limitée à un moyen particulier pour donner au dispositif sa capacité de déformation (paroi souple, paroi articulée, lignes de pliages, etc).

Le plus souvent on profitera en combinaison de la souplesse des panneaux et de quelques lignes de pliage ; ainsi les pliages 3 et 4 peuvent être dues à des lignes de pliage ou à une capacité de déformation du panneau central (D) qui dans ce cas constitue un seul panneau avec les panneaux (C) et (E).

## Revendications

1. Dispositif anti-pique pour manipuler avec sécurité une aiguille d'injection présentant une branche distale perforante (17), ce dispositif comprenant une succession de panneaux (A-F) articulés de proche en proche par des lignes de pliage (1-5), ces panneaux comprenant deux plaquettes (F,D), l'une (F) des plaquettes dite plaquette porte-aiguille étant apte à porter l'aiguille et l'autre plaquette (D) dite plaquette de base étant apte à être appliquée contre la peau autour du point d'injection, lesdites plaquettes présentant des trous respectifs (15,16) pour laisser passer la branche distale (17) de l'aiguille, ces plaquettes étant reliées par les autres panneaux (B,C,E,A) en sorte que le dispositif puisse passer d'une configuration où lesdits trous sont alignés à une configuration où les panneaux entourent un espace suffisant pour que, lors de l'extraction de l'aiguille hors de la peau, ladite branche distale de l'aiguille soit escamotable dans ledit espace au fur et à mesure de son extraction, **caractérisé en ce que** la plaquette porte-aiguille (F) porte deux oreilles latérales (9,10) relevables pour pouvoir être mises en vis-à-vis l'une de l'autre et servir à la manipulation du dispositif.

2. Dispositif selon la revendication 1, dont l'un des autres panneaux est un panneau dit d'extrémité (A) apte à être rabattu et fixé sur la plaquette porte-aiguille (F), et qui présente deux perforations allongées (7,8) dans lesquelles peuvent passer lesdites oreilles latérales (9,10) lorsque ces oreilles ont été mises en vis-à-vis l'une de l'autre, et que le panneau d'extrémité est rabattu sur la plaquette porte-aiguille.

3. Dispositif selon la revendication 2 dans lequel ledit panneau d'extrémité (A) présente une gorge (6) pour recevoir une branche proximale (18,19) de l'aiguille qui forme un coude avec ladite branche distale (17) de l'aiguille.

4. Dispositif selon la revendications 2 ou 3 constitué d'une paroi unique qui présente des lignes de pliage (1-5) partageant la paroi en ladite succession de panneaux en sorte que la plaquette porte-aiguille (F) soit rabattable sur la plaquette de base (D) et que le panneau d'extrémité (A) soit ensuite rabattable sur la plaquette porte-aiguille.

5. Dispositif selon la revendication 4 dans lequel ladite paroi est une feuille semi-rigide venue de découpage d'un flan en matériau de synthèse.

6. Dispositif selon la revendication 4 ou 5 dans lequel la plaquette de base (D) comporte deux ailettes latérales (11,12) qui facilitent son application sur la peau et disposées en sorte que lesdites oreilles (9,10) de la plaquette porte-aiguille (F) viennent s'appliquer sur ces ailettes lorsque la plaquette porte-aiguille est rabattue contre la plaquette de base (D).

## Patentansprüche

1. Antistechvorrichtung zum sicheren Handhaben einer Injektionsnadel, die eine distale, perforierende Abzweigung (17) aufweist, wobei diese Vorrichtung eine Folge von Platten (A - F) umfaßt, die durch Biegelinien (1 - 5) aufeinanderfolgend gelenkig aneinandergesetzt sind, diese Platten zwei Plättchen (F, D) umfassen, von denen das eine (F) der Plättchen, genannt Nadelträgerplättchen, dazu geeignet ist, die Nadel zu tragen, und das andere Plättchen (D), genannt Grundplättchen, dazu geeignet ist, gegen die Haut um den Einstichpunkt angelegt zu werden, wobei die genannten Plättchen jeweils Löcher (15,16) aufweisen, um die distale Abzweigung (17) der Nadel hindurchzulassen, und wobei diese Plättchen mit den anderen Platten (B, C, E, A) derart verbunden sind, daß die Vorrichtung aus einer Konfiguration, in der die Löcher ausgerichtet sind, in eine Konfiguration übergehen kann, in der die Platten einen Raum umgeben, der ausreicht, damit beim Ausziehen der Nadel aus der Haut die genannte, distale Abzweigung der Nadel im Verlauf ihres Ausziehens in den genannten Raum einziehbar ist, **dadurch gekennzeichnet, daß** das Nadelträgerplättchen (F) zwei seitliche, anhebbare Ohren (9, 10) trägt, die einander gegenüberliegend angeordnet werden können und zum Handhaben der Vorrichtung dienen können.

2. Vorrichtung nach Anspruch 1, bei der eine der anderen Platten eine Platte ist, die Endplatte (A) genannt ist, die dazu geeignet ist, auf das Nadelträgerplättchen (F) zurückgeschlagen und dort befestigt zu werden, und die zwei längliche Perforierungen (7, 8) aufweist, in denen die genannten, seitlichen Ohren (9, 10) hindurchtreten können, wenn diese Ohren einander gegenüberliegend angeordnet werden, und daß die Endplatte auf die Nadelträgerplatte zurückgeschlagen ist.

3. Vorrichtung nach Anspruch 2, worin die genannte Endplatte (A) eine Nut (6) zur Aufnahme einer proximalen Abzweigung (18, 19) der Nadel aufweist, die einen Knick zur distalen Abzweigung (17) der Nadel bildet.

4. Vorrichtung nach Anspruch 2 oder 3, gebildet aus einer einzigen Wand, die Biegelinien (1 - 5) aufweist, die die Wand in die genannte Folge von Platten aufteilt, und zwar derart, daß das Nadelträgerplättchen (F) auf das Grundplättchen (D) zurückschlagbar ist, und daß die Endplatte (A) nachfolgend auf das Nadelträgerplättchen zurückschlagbar ist.

5. Vorrichtung nach Anspruch 4, worin die genannte Wand ein halbstarrer Bogen ist, der dem Zuschnitt einer Kunststoffplatine entstammt.

6. Vorrichtung nach Anspruch 4 oder 5, in welcher das Grundplättchen (D) zwei seitliche Flügel (11, 12) aufweist, die ihr Aufsetzen auf die Haut erleichtern und derart angebracht sind, daß die genannten Ohren (9, 10) des Nadelträgerplättchens (F) zur Anlage auf diesen Flügeln gelangen, wenn das Nadelrägerplättchen gegen das Grundplättchen (D) zurückgeschlagen ist.

## Claims

1. Anti-pricking device for the safe handling of an injection needle having a piercing distal branch (17), this device comprising a succession of panels (A-F) articulated to one another by fold lines (1-5), these panels comprising two plates (F, D), one (F) of the plates, known as the needle-carrying plate, being able to carry the needle and the other plate (D), known as the base plate, being suitable for being pressed against the skin around the point of injection, the said plates having respective holes (15,16) to allow the distal branch (17) of the needle through, these plates being connected by the other panels (B, C, E, A) in such a way that the device can change from a configuration in which the said holes are aligned to a configuration in which the panels surround enough space that, as the needle is extracted from the skin, the said distal branch of the needle can be retracted into the said space as it is extracted, **characterized in that** the needle-carrying plate (F) bears two lateral lugs (9, 10) which can be raised so that they can be brought to face one another and be used to handle the device.

2. Device according to Claim 1, of which one of the other panels is a panel known as an end panel (A) able to be folded down and fixed onto the needle-carrying plate (F) and which has two elongate perforations (7, 8) into which the said lateral lugs (9, 10) can be passed when these lugs have been brought to face one another, and the end panel is folded down onto the needle-carrying plate.

3. Device according to Claim 2, in which the said end panel (A) has a recess (6) to accept a proximal branch (18, 19) of the needle which forms an elbow with the said distal branch (17) of the needle.

4. Device according to Claim 2 or 3, consisting of a single wall which has fold lines (1-5) dividing the wall into the said succession of panels so that the needle-carrying plate (F) can be folded down onto the base plate (D) and so that the end panel (A) can then be folded down onto the needle-carrying plate.

5. Device according to Claim 4, in which the said wall is a semi-rigid sheet cut from a blank of synthetic material.

6. Device according to Claim 4 or 5, in which the base plate (D) comprises two lateral wings (11, 12) which make it easier to apply to the skin and are arranged in such a way that the said lugs (9, 10) of the needle-carrying plate (F) come to rest against these wings when the needle-carrying plate is folded down against the base plate (D).
